# EUROPEAN PATENT APPLICATION

(11) **EP 2 578 170 A1**
(43) Date of publication of application: **10.04.2013**
(21) Application number: 11789523.5
(22) Date of filing: 31.03.2011
(51) Int. Cl.: A61B 17/28, A61B 19/00, B25J 17/00, F16C 1/10

(54) **DRIVING FORCE TRANSMITTING MECHANISM AND MANIPULATOR SYSTEM**

(30) Priority: 31.05.2010 JP 2010125152
(71) Applicant: OLYMPUS CORPORATION, Shibuya-ku Tokyo 151-0072 (JP)
(72) Inventor: IIDA, Masatoshi, Hachioji-shi Tokyo 192-8512 (JP)
(74) Representative: Schicker, Silvia
(86) International application number: PCT/JP2011/058181
(87) International publication number: WO 2011/152113

(57) **Abstract**

A driving force transmission mechanism (1) for transmitting a driving force from a driving source has the following arrangement. That is, the driving force transmission mechanism (1) includes a linear or rod-shaped flexible manipulation wire (95, 96), an inner pipe (2) serving as a flexible guide member in which the manipulation wire (95, 96) is inserted, an outer pipe (3) provided radially outside the inner pipe (2) and configured to suppress deformation of the inner pipe (2).

## Description

### Technical Field

The present invention relates to a driving force transmission mechanism for transmitting a driving force from a driving source and a manipulator system comprising the driving force transmission mechanism.

### Background Art

As a technique concerning a driving force transmission mechanism and a manipulator system, Jpn. UM Appln. KOKAI Publication No. 6-81503, for example, discloses the following technique. That is, Jpn. UM Appln. KOKAI Publication No. 6-81503 discloses, as a driving force transmission mechanism, an endoscope bending apparatus comprising a bending manipulation wire serving as a flexible transmission member used to bend a bending portion connected to the distal end side of a flexible tube, and a flexible guide tube which is formed from a closely wound coil and guides the bending manipulation wire.

When the technique disclosed in Jpn. UM Appln. KOKAI Publication No. 6-81503 is applied, the guide tube formed from a closely wound coil may buckle due to the tensile force of the bending manipulation wire at the time of bending manipulation. If buckling occurs, the bending length of the bending portion is lost by an amount corresponding to the change in the length of the guide tube caused by the buckling.

Considering the problem that arises due to the buckling, Jpn. Pat. Appln. KOKAI Publication No. 6-300975 discloses a technique of causing a tubular member formed from a superelastic pipe hard to buckle to guide a shape memory alloy wire serving as a power transmission member that performs a flexible tube bending manipulation.

However, even when the technique disclosed in Jpn. Pat. Appln. KOKAI Publication No. 6-300975 is applied, a tensile force applied to the shape memory alloy wire compresses/deforms (undulates) the tubular member formed from a superelastic pipe. The superelastic pipe radially deforms, impeding transmission of the tensile force (the power transmission efficiency is reduced).

### Disclosure of Invention

The present invention has been made in consideration of the above-described situations, and has as its object to provide a driving force transmission mechanism that does not reduce the transmission efficiency of a power transmission member (does not degrade the transmission characteristic), and a manipulator system comprising the driving force transmission mechanism.

In order to achieve the above object, according to a first aspect of the invention, there is provided a driving force transmission mechanism including a linear or rod-shaped flexible transmission member configured to transmit a driving force from a driving source, and a guide member that is a flexible tubular member in which the transmission member is inserted, characterized by comprising:
a constraint member provided radially outside the guide member and configured to suppress deformation of the guide member.

In order to achieve the above object, according to a second aspect of the invention, there is provided a manipulator system characterized by comprising:
a driving force transmission mechanism configured to transmit a driving force from a driving source;
a bending tube having a plurality of joint portions to be operated by the driving force transmitted by the driving force transmission mechanism;
a manipulation unit configured to operate the joint portions; and
a control unit configured to control the operation of the joint portions based on the manipulation of the manipulation unit,
the driving force transmission mechanism comprising:
   a linear or rod-shaped flexible transmission member configured to transmit the driving force;
   a guide member that is a flexible tubular member in which the transmission member is inserted; and
   a constraint member provided radially outside the guide member and configured to suppress deformation of the guide member.

### Brief Description of Drawings

FIG. 1 is a view showing an example of the arrangement of a manipulator system to which a driving force transmission mechanism according to an embodiment of the present invention is applied.
FIG. 2 is a view showing an example of the arrangement of the insertion portion of a manipulator.
FIG. 3A is a side sectional view of the bending portion of the insertion portion.
FIG. 3B is a side sectional view of the driving force transmission mechanism that constitutes the bending portion.
FIG. 4 is a sectional view of the driving force transmission mechanism taken along line A-A' in FIG. 3.
FIG. 5 is a sectional view of a driving force transmission mechanism according to the first modification taken along a portion corresponding to line A-A' in FIG. 3.
FIG. 6 is a side view showing an example of the arrangement of a driving force transmission mechanism according to the second modification.
FIG. 7 is a side sectional view showing an example of the arrangement of a driving force transmission mechanism according to the third modification.

### Mode for Carrying Out the Invention

A driving force transmission mechanism according to an embodiment of the present invention and a manipulator system including the driving force transmission mechanism will now be described. Note that in this embodiment, the manipulator system is assumed to be a treatment manipulator system used in, for example, an endoscopic surgical operation.

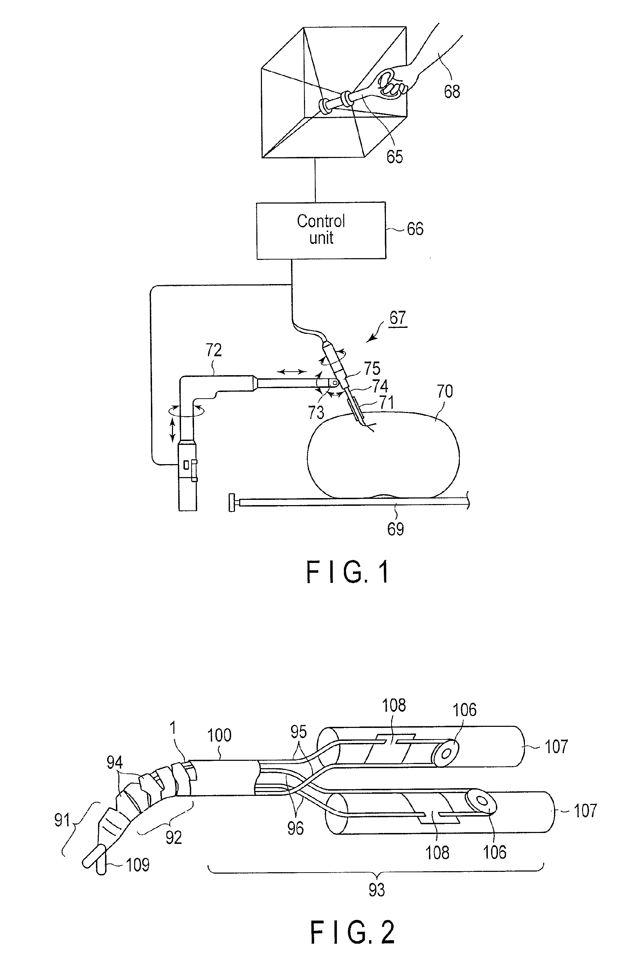

FIG. 1 is a view showing an example of the arrangement of a manipulator system to which a driving force transmission mechanism according to this embodiment is applied. As shown in FIG. 1, the manipulator system comprises a manipulation input device 65 serving as a manipulation unit, a motor control unit 66 serving as a control unit, and a manipulator 67.

The manipulation input device 65 reads information about the position, orientation, and grip of a hand 68 of an operator, and outputs these pieces of information to the motor control unit 66.

The motor control unit 66 controls the manipulator 67 based on the information about the position, orientation, and grip of the hand 68 of the operator output from the manipulation input device 65.

The manipulator 67 is used to treat a morbid portion of a patient 70. An insertion portion 74 of the manipulator 67 is inserted into a trocar 71 inserted in the body of the patient 70 fixed on an operating table 69.

More specifically, the manipulator 67 includes a robot arm 72 that has 3 degrees of freedom and operates based on a cylindrical coordinate system, a universal joint 73 that has 2 degrees of freedom and operates in the vertical direction and rotational direction, the insertion portion 74 inserted into the body of the patient 70, and a holder 75 that rotates the insertion portion 74.

The insertion portion 74 of the manipulator 67 will be described below in detail. FIG. 2 is a view showing an example of the arrangement of the insertion portion 74 of the manipulator 67.

The insertion portion 74 of the manipulator 67 includes a distal end 91 including a forceps 109, a bending portion 92 serving as a joint portion having a plurality of joints, and a driven portion 93 including an insertion pipe 100.

The bending portion 92 includes bending tubes 94 each serving as a joint that bends through 2 degrees of freedom and operates vertically and horizontally, two manipulation wires 95 each serving as a flexible linear transmission member used to vertically manipulate the bending tube 94, and two manipulation wires 96 each serving as a flexible linear transmission member used to horizontally manipulate the bending tube 94.

Each of the manipulation wires 95 and 96 has one end connected and fixed to the bending tube 94 at the extreme tip and/or the distal end 91 in a state in which the manipulation wires are circumferentially spaced apart from each other at an interval of 90° in the bending tube 94 at the extreme tip and/or the distal end 91. In addition, each of the manipulation wires 95 and 96 has other end connected and fixed to a ball screw nut 108 of a linear-acting actuator 107 including a pulley 106 and the ball screw nut 108. The manipulation wires 95 and 96 are flexible.

Note that each of the manipulation wires 95 and 96 is inserted into an inner pipe serving as a guide member, and details will be described later with reference to the accompanying drawings. The inner pipe is inserted into an outer pipe serving as a constraint member.

When the operator treats the patient 70 by manipulating the manipulation input device 65 and operating the manipulator 67, the respective portions of the manipulator 67 operate as follows. That is, pieces of information about the position, orientation, and grip of the hand 68 of the operator which are output from the manipulation input device 65 are input to the motor control unit 66. The motor control unit 66 converts the information into the manipulated variable of the robot arm 72, the rotation amount of the insertion portion 74 by the universal joint 73, the bending amount of the bending portion 92 in the insertion portion 74, and the open/close angle of the forceps 109.

In the driven portion 93, the ball screw nuts 108 move forward and backward as a motor (not shown) serving as a driving source rotates. The manipulation wires 95 and 96 are differentially driven in accordance with the forward/backward movement, and the bending portion 92 bends vertically and horizontally. Similarly, the forceps 109 opens and closes as the motor (not shown) rotates.

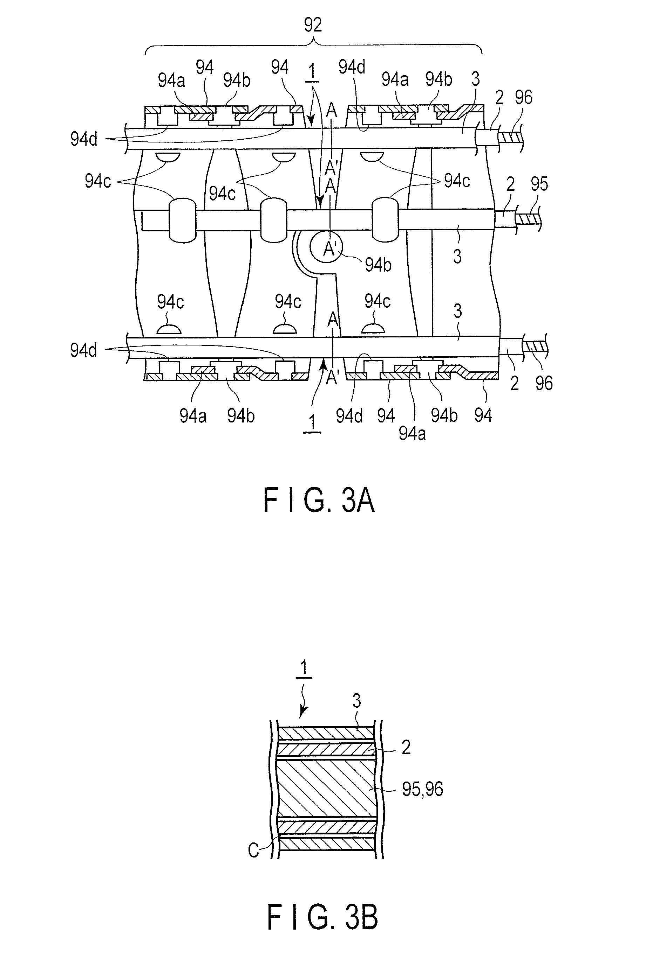

A driving force transmission mechanism according to this embodiment, which is applied to the bending portion 92, will be explained below. FIG. 3A is a side sectional view of the bending portion 92.

The bending portion 92 comprises the plurality of bending tubes 94 connected to each other, inner pipes 2 serving as the guide members of the manipulation wires 95 and 96, and outer pipes 3 each serving as a constraint member (deformation prevention member) that suppresses (stops or regulates) radial deformation of the inner pipe 2. A driving force transmission mechanism 1 is formed from the manipulation wire 95 or 96, the inner pipe 2, and the outer pipe 3.

The plurality of bending tubes 94 are pivotally connected by pins 94b at connection portions 94a in a state in which the connection portions 94a of the bending tubes 94 adjacent to each other are arranged while being circumferentially shifted by 90° from each other. When the bending tubes 94 are connected in this way, the whole bending portion 92 can bend vertically and horizontally.

The inner surface of each bending tube 94 is provided with a plurality of holding portions 94c to be described below. That is, each of the plurality of holding portions 94c serves as a member that holds the driving force transmission mechanism 1 while allowing its axial movement. The holding portions 94c are circumferentially attached to the inner surfaces of the bending tubes 94 at an interval of 90° and at a predetermined axial distance.

More specifically, each holding portion 94c has a through hole 94d whose diameter is slightly larger than the outer diameter of the outer pipe 3. The axial movement of each driving force transmission mechanism 1 is allowed via the through holes 94d.

A pair of driving force transmission mechanisms 1 corresponding to the pair of manipulation wires 95 and a pair of driving force transmission mechanisms 1 corresponding to the pair of manipulation wires 96 are circumferentially located at an interval of 90° relative to the bending tubes 94.

Note that in this example, each driving force transmission mechanism 1 disposed in the bending portion 92 extends into the insertion pipe 100.

The driving force transmission mechanism 1 will be described below in detail. The driving force transmission mechanism 1 is provided in correspondence with each manipulation wire included in the manipulation wires 95 and 96 that are pairs of manipulation wires. Hence, a total of four driving force transmission mechanisms are disposed in this example.

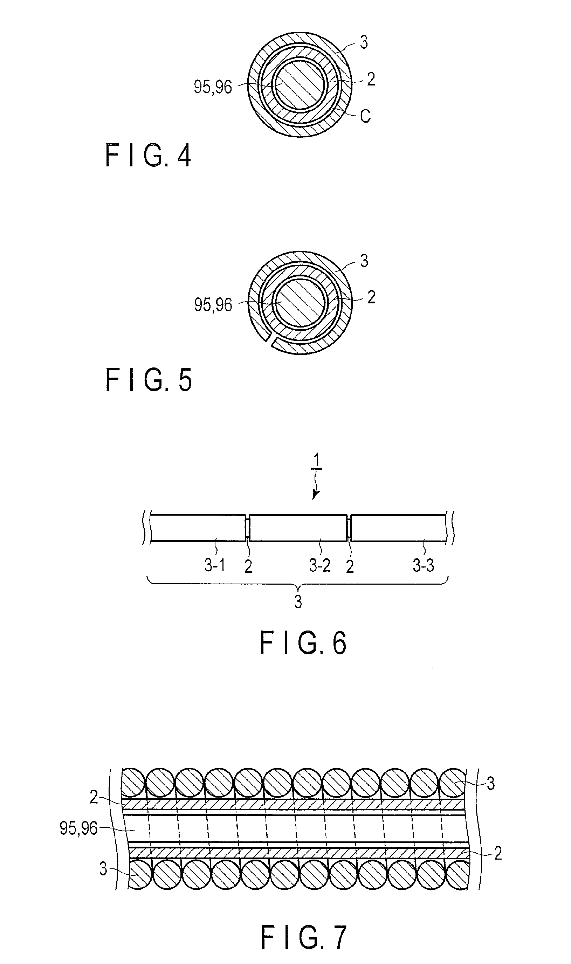

FIG. 3B is a side sectional view of the driving force transmission mechanism 1 applied to the bending portion 92. FIG. 4 is a sectional view of the driving force transmission mechanism 1 taken along line A-A' in FIG. 3.

The inner pipe 2 is a guide member that guides the manipulation wire 95 or 96. More specifically, the inner pipe 2 is a member with the following structure.
(1) The inner pipe 2 is a flexible tubular member having an almost annular section.
(2) The manipulation wire 95 or 96 is inserted into the inner pipe 2. In other words, the inner pipe 2 is disposed radially outside the manipulation wire 95 or 96.
(3) As the material of the inner pipe 2, a flexible metal material is suitable. An example is Ni-Ti.
(4) The inner surface of the inner pipe 2 is preferably polished to reduce friction with the manipulation wire 95 or 96 caused by the operation of the manipulation wire 95 or 96.

The outer pipe 3 is a constraint member that constrains the inner pipe 2 so as to suppress (stop or regulate) radial deformation of the inner pipe 2. More specifically, the outer pipe 3 is a member with the following structure.
(1) The outer pipe 3 is a flexible tubular member having an almost annular section.
(2) The inner pipe 2 is inserted into the outer pipe 3 such that a clearance C is formed between the outer pipe 3 and the inner pipe 2. More specifically, the clearance C allows the inner pipe 2 to axially move in the outer pipe 3.
(3) The outer diameter of the inner pipe 2 almost equals the inner diameter of the outer pipe 3.
(4) As the material of the outer pipe 3, a flexible (or elastic) metal material is suitable. Examples are SUS and Ni-Ti.

Note that in the insertion pipe 100 as well, each driving force transmission mechanism 1 can be held using the holding portions 94c, like the arrangement in the bending portion 92, or each driving force transmission mechanism 1 may simply be inserted into the insertion pipe 100 without using the holding portions 94c. Conversely, if the space in the bending portion 92 is small, the holding portions 94c need not be provided in the bending portion 92.

In this example, the inner pipe 2 is disposed from the distal end of the bending portion 92 up to the proximal portion of the insertion pipe 100. The outer pipe 3 is provided throughout the length of the inner pipe 2. That is, each driving force transmission mechanism 1 is disposed from the distal end of the bending portion 92 up to the proximal portion of the insertion pipe 100.

As described above, the manipulation wires 95 and 96, the inner pipe 2, and the outer pipe 3 are flexible (or elastic), and bend in accordance with the manipulation of the manipulation input device 65 by the hand 68 of the operator.

In the related art, the inner pipe 2 that has bent at the time of a bending operation radially deforms (for example, buckles), and the transmission efficiency of the manipulation wire 95 or 96 in the inner pipe 2 is reduced accordingly (the transmission characteristic degrades).

On the other hand, according to the driving force transmission mechanism 1 of this embodiment, the outer pipe 3 suppresses (stops or regulates) radial deformation of the inner pipe 2 at the time of a bending operation. This makes it possible to prevent the decrease in driving force transmission efficiency (the degradation in the transmission characteristic) due to the state of being bent.

As described above, according to this embodiment, it is possible to provide a driving force transmission mechanism that does not reduce the transmission efficiency of the power transmission member (does not degrade the transmission characteristic) at the time of a bending operation, and a manipulator system comprising the driving force transmission mechanism.

More specifically, according to the driving force transmission mechanism and the manipulator system of this embodiment, for example, the following particular effects can be obtained.
· The outer pipe 3 that constrains radial deformation of the inner pipe 2 is radially provided outside the inner pipe 2, which serves as the guide member of the manipulation wire 95 or 96. Hence, even when a tensile force is applied to the inner pipe 2 at the time of manipulation of the manipulation wire 95 or 96, the inner pipe 2 is constrained by the outer pipe 3, and compression/deformation of the inner pipe 2 (radial deformation) is thus suppressed. That is, at the time of a bending operation, an external force that reduces the transmission efficiency does not act on the manipulation wire 95 or 96.
· The change in the path length of the manipulation wire 95 or 96 can be prevented. It is therefore possible to prevent an increase in the contact area between the inner pipe 2 and the manipulation wire 95 or 96 caused by the compression/deformation (undulation) of the inner pipe 2 and suppress attenuation by the friction between the inner surface of the inner pipe 2 and the manipulation wire 95 or 96 caused by the radial deformation of the inner pipe 2. This makes it possible to obtain a satisfactory transmission characteristic with little hysteresis characteristic.
· Not only the radial deformation caused by compression/deformation of the inner pipe 2 when a tensile force is applied to the manipulation wire 95 or 96 but also radial deformation of the inner pipe 2 caused by another factor is suppressed.
· When the inner pipe 2 and the outer pipe 3 are made of a metal material, the strength of the driving force transmission mechanism 1 increases.
· The outer diameter of the inner pipe 2 almost equals the inner diameter of the outer pipe 3. In addition, the clearance C is provided to make the inner pipe 2 axially movable in the outer pipe 3. Hence, the inner pipe 2 can axially move in the outer pipe 3. This further enhances the effect of suppressing the radial deformation of the inner pipe 2.
· Since the outer pipe 3 is provided throughout the length of the inner pipe 2, the radial deformation of the inner pipe 2 can be suppressed throughout its length.

In the above-described example, the outer pipe 3 is made of a metal material. However, the outer pipe 3 may be made of, for example, a hard resin if it can regulate the radial deformation of the inner pipe 2.

In the above-described example, the outer pipe 3 is formed into an almost annular section. However, the outer pipe 3 may be formed into an almost polygonal section. In this case, the inner pipe 2 preferably has the same sectional shape as that of the outer pipe 3.

The outer pipe 3 is provided throughout the length of the inner pipe 2. However, the outer pipe 3 may be provided at part (partially) of the inner pipe 2. For example, the outer pipe 3 may be provided on the inner pipe 2 only at a portion that especially readily bends or bends greatly.

In the above-described example, a wire (manipulation wire 95 or 96) is used as a transmission member. However, a wire made of, for example, a shape memory alloy or a wire or rod formed as a simple rod-shaped member may be used.

In the above-described example, the manipulator 67 comprising the forceps 109 has been exemplified as a manipulator. However, this embodiment is not limited to this and is also applicable to, for example, an observation manipulator comprising an endoscope. That is, this embodiment is applicable to various kinds of manipulators including a treatment manipulator and an observation manipulator (endoscope).

This embodiment is also applicable not only to a medical manipulator system but also to an industrial manipulator system or a manual manipulator system, as a matter of course.

The present invention has been described above based on an embodiment. However, the present invention is not limited to the above-described embodiment, and various modifications and applications can be made without departing from the scope of the invention, as a matter of course.

### «First Modification»

The first modification of the driving force transmission mechanism and the manipulator system according to the above-described embodiment will be described below. To avoid a repetitive explanation, the difference from the driving force transmission mechanism and the manipulator system according to the embodiment will be described. In the above embodiment, the outer pipe 3 is formed as a tubular member having an almost annular section. In the first modification, the outer pipe 3 has the structure described below.

FIG. 5 is a sectional view of a driving force transmission mechanism according to the first modification taken along a portion corresponding to line A-A' in FIG. 3. As shown in FIG. 5, in the first modification, a notch (slit) is axially formed in the outer surface of the outer pipe 3 so that the outer pipe 3 has a C-shaped section.

As described above, according to the first modification, it is possible to provide a driving force transmission mechanism and a manipulator system which have the same effects as those of the driving force transmission mechanism and the manipulator system according to the embodiment.

### «Second Modification»

The second modification of the driving force transmission mechanism and the manipulator system according to the above-described embodiment will be described below. To avoid a repetitive explanation, the difference from the driving force transmission mechanism and the manipulator system according to the embodiment will be described. In the above embodiment, the outer pipe 3 is formed as one tubular member (one outer pipe 3 in one driving force transmission mechanism 1) corresponding to one manipulation wire 95 or 96. In the second modification, the outer pipe 3 has the structure described below.

FIG. 6 is a side view showing an example of the arrangement of a driving force transmission mechanism according to the second modification. As shown in FIG. 6, in the second modification, the outer pipe 3 is divided into a plurality of parts in the longitudinal direction of the inner pipe 2.

In other words, a plurality of short pipes 3-1, 3-2, 3-3, ... are disposed in series for the inner pipe 2, as shown in FIG. 6, thereby forming the outer pipe 3 by the plurality of short pipes 3-1, 3-2, 3-3, .... More specifically, when the total length of the outer pipe 3 is, for example, 1 m, the length of each of the short pipes 3-1, 3-2, 3-3, ... is about 5 mm.

The short pipes 3-1, 3-2, 3-3, ... each of which is much shorter relative to the total length of the outer pipe 3 are disposed in series while being connected in a row. In this case, although each of the short pipes 3-1, 3-2, 3-3, ... is not flexible (or elastic), the whole outer pipe 3 can sufficiently flex (bend).

As described above, according to the second modification, it is possible to provide a driving force transmission mechanism and a manipulator system which have the same effects as those of the driving force transmission mechanism and the manipulator system according to the embodiment, and also offer a wide choice of materials for the outer pipe 3.

### «Third Modification»

The third modification of the driving force transmission mechanism and the manipulator system according to the above-described embodiment will be described below. To avoid a repetitive explanation, the difference from the driving force transmission mechanism and the manipulator system according to the embodiment will be described. In the above embodiment, the outer pipe 3 is formed as one flexible tubular member having an almost annular section. In the third modification, the outer pipe 3 has the structure described below.

FIG. 7 is a side sectional view showing an example of the arrangement of a driving force transmission mechanism according to the third modification. As shown in FIG. 7, in the third modification, the outer pipe 3 is formed as a closely wound coil. That is, a tubular member formed from a closely wound coil is used as the outer pipe 3.

As described above, according to the third modification, it is possible to provide a driving force transmission mechanism and a manipulator system which have the same effects as those of the driving force transmission mechanism and the manipulator system according to the embodiment.

The above-described embodiment incorporates inventions of various stages, and various inventions can be extracted by appropriately combining a plurality of disclosed constituent elements. For example, if it is possible to solve the problem described in the paragraph of "problem to be solved by the invention" and obtain the effects described in the paragraph "advantages of the invention" even when several constituent elements are removed from all constituent elements of the embodiment, the arrangement without these constituent elements can also be extracted as an invention.

## Claims

1. A driving force transmission mechanism including a linear or rod-shaped flexible transmission member configured to transmit a driving force from a driving source, and a guide member that is a flexible tubular member in which the transmission member is inserted, **characterized by** comprising:
a constraint member provided radially outside the guide member and configured to suppress deformation of the guide member.

2. The driving force transmission mechanism according to claim 1, **characterized in that**
an outer diameter of the guide member substantially equals an inner diameter of the constraint member, and
a clearance that allows the guide member to axially move in the constraint member is provided between the constraint member and the guide member.

3. The driving force transmission mechanism according to claim 2, **characterized in that** the constraint member includes a flexible or elastic tubular member.

4. The driving force transmission mechanism according to claim 2, **characterized in that** the constraint member is formed by disposing a plurality of tubular members in series, and the whole constraint member is flexible.

5. The driving force transmission mechanism according to claim 2, **characterized in that** the constraint member includes a member formed from a closely wound coil and having a tubular shape.

6. A manipulator system **characterized by** comprising:
a driving force transmission mechanism configured to transmit a driving force from a driving source;
a bending tube having a plurality of joint portions to be operated by the driving force transmitted by the driving force transmission mechanism;
a manipulation unit configured to operate the joint portions; and
a control unit configured to control the operation of the joint portions based on the manipulation of the manipulation unit,
the driving force transmission mechanism comprising:
a linear or rod-shaped flexible transmission member configured to transmit the driving force;
a guide member that is a flexible tubular member in which the transmission member is inserted; and
a constraint member provided radially outside the guide member and configured to suppress deformation of the guide member.
